# EUROPÄISCHE PATENTSCHRIFT

(11) **EP 0 544 266 B1**
(45) Veröffentlichungstag und Bekanntmachung des Hinweises auf die Patenterteilung: **25.09.1996**
(21) Anmeldenummer: 92120115.8
(22) Anmeldetag: 25.11.1992
(51) Int. Cl.: C07D 207/44

(54) **Verfahren zur Herstellung von Bismaleinimid-Derivaten**
Process for the preparation of bismaleimides
Procédé pour la préparation des bismaléimides

(30) Priorität: 28.11.1991 CH 3486/91
(43) Veröffentlichungstag der Anmeldung: 02.06.1993
(73) Patentinhaber: LONZA AG, CH-3945 Gampel/Wallis (CH)
(72) Erfinder: Hardt, Peter, Dr., Visp (Kanton Wallis) (CH); Völker, Theodor, Dr., Reinach (Kanton Baselland) (CH)
(74) Vertreter: Weinhold, Peter, Dr.

(56) Entgegenhaltungen:
- EP-A- 0 334 497
- EP-A- 0 387 381
- EP-A- 0 393 713
- GB-A- 2 043 054
- PATENT ABSTRACTS OF JAPAN vol. 10, no. 269 (C-372)(2325) 12. September 1986
- PATENT ABSTRACTS OF JAPAN vol. 7, no. 3 (C-143)(1148) 7. Januar 1983

## Beschreibung

Die Erfindung betrifft ein neues Verfahren zur Herstellung von Bismaleinimid-Derivaten der allgemeinen Formel 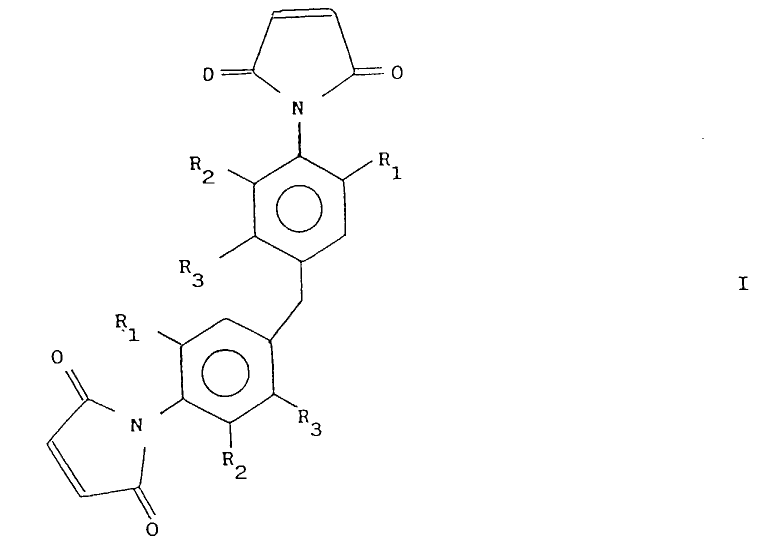 worin R₁ und R₂ gleich oder verschieden sind und eine C₁-C₄-Alkylgruppe, verzweigt oder unverzweigt, bedeuten und R₃ ein Halogen- oder Wasserstoffatom bedeutet, ausgehend von Maleinsäureanhydrid und einem Methylen-bis-anilin.

Diese Bismaleinimid-Derivate werden beispielsweise zur Herstellung von hitzebeständigen Bismaleinimid-Harzen verwendet (JP-A 61 093 159).

Aus der EP-A-0 393 713 ist es bekannt, N-substituierte Maleinimide durch Erhitzen von N-substituierten Maleaminsäuremonoestern in Gegenwart eines sauren Katalysators durch Abspaltung des Alkohols vom Ester herzustellen.

In der EP-A-0 334 497 wird ein Mehrstufenverfahren zur Herstellung von Maleinimid beschrieben. Hierbei werden in einem ersten Schritt Maleinsäureanhydrid und ein primäres aromatisches Amin in einem Molverhältnis von weniger als 1 unter Wärmeeinwirkung zu einem wasserunlöslichen oder mit Wasser nicht mischbaren inerten organischen Lösungsmittel gegeben, das einen sauren Katalysator enthält. Anschließend wird dem Reaktionssystem in einem zweiten Schritt weiteres Maleinsäureanhydrid in einer solchen Menge zugegeben, daß die Gesamtmenge an Maleinsäureanhydrid zu Amin ein Molverhältnis von 1 überschreitet.

Die GB-A-2 043 054 offenbart ein Verfahren zur Herstellung von N-(2-Methyl-1-naphthyl)-maleinimid. Dabei werden 2-Methyl-1-naphthylamin und Maleinsäureanhydrid in einem Molverhältnis zwischen 1:1 und 1:1,2 in Gegenwart eines inerten organischen Lösungsmittels und eines sauren Katalysators, beispielsweise von Methansulfonsäure, auf eine Temperatur erhitzt, bei der das Wasser aus der flüssigen Mischung verdampft und das Erhitzen wird anschließend solange fortgesetzt, bis das Destillat wasserfrei ist.

Die EP-A-0 387 381 beschreibt ein Mehrstufenverfahren zur Herstellung von Bismaleinimiden. Dabei wird beispielsweise in einem ersten Schritt Maleinsäureanhydrid mit 4,4'-Diaminodiphenylmethan in Aceton umgesetzt. Die so erhaltene N,N'-4,4'-Diphenylmethanbismaleaminsäure wird dann mit einem Orthoester verestert und schließlich in einem weiteren Schritt zum entsprechenden Bismaleinimid cyclisiert.

Eine weitere bekannte Ausführungsform zur Herstellung von Bismaleinimid-Derivaten ist beispielsweise in der JP-A 57 159 764 (JP-B 02 058 267) beschrieben.

Bei diesem Verfahren wird ein Methylenbis[N-(monoalkyl)phenylen]maleinimid], ausgehend von Maleinsäureanhydrid und einem Methylen-bis-anilin, in Gegenwart eines sauren Katalysators hergestellt. Um ein reines Produkt mit guter Ausbeute zu erhalten, wird bei diesem Verfahren zwingend als Lösungsmittel ein Gemisch von einem halogenierten Kohlenwasserstoff mit einem aprotischen polaren Lösungsmittel verwendet. Aus dem Vergleichsbeispiel 2 (Seite 10) der genannten Anmeldung geht hervor, dass wenn Dichlorethan durch Toluol im Lösungsmittelgemisch ausgetauscht wird,sowohl die Reinheit von 93% auf 67% als auch die Ausbeute des gewünschten Endproduktes von 95% auf 88% reduziert wird. Der Austausch von Dichlorethan durch Xylol reduziert sogar sowohl die Reinheit von 95% auf 53% als auch die Ausbeute des gewünschten Endproduktes von 95% auf 64%.

Ein grosser Nachteil dieses Verfahrens besteht darin, dass die umweltbelastenden halogenierten Kohlenwasserstoffe als Lösungsmittel eingesetzt werden müssen, um ein reines Endprodukt in guter Ausbeute zu erhalten.

Die Aufgabe der Erfindung war, diesen Nachteil zu beseitigen und ein ökologisches Verfahren zur Herstellung von Bismaleinimid-Derivaten zur Verfügung zu stellen.

Diese Aufgabe wurde mit dem erfindungsgemässen Verfahren gemäss Patentanspruch 1 gelöst.

Erfindungsgemäss wird das Verfahren derart durchgeführt, dass man in der ersten Stufe Maleinsäureanhydrid der Formel 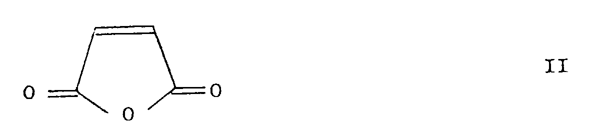 mit einem Methylen-bis-anilin der allgemeinen Formel 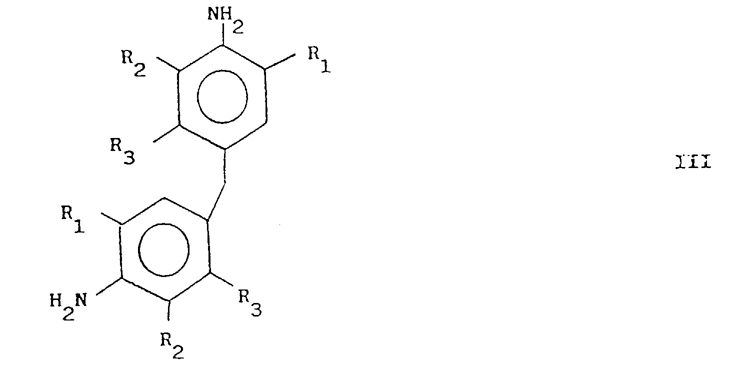 worin R₁, R₂ und R₃ die genannte Bedeutung haben, in einem unhalogenierten aromatischen Kohlenwasserstoff als Lösungsmittel in die entsprechende Amidsäure überführt und diese Amidsäure, ohne Isolierung, in der zweiten Stufe in Gegenwart eines sauren Katalysators in das Endprodukt gemäss der allgemeinen Formel I überführt.

Die Umsetzung in der ersten Stufe erfolgt zweckmässig mit einem Methylen-bis-anilin, worin
R₁ = R₂ = Methyl, Ethyl- oder Isopropyl- und
R₃ ein Wasserstoffatom bedeuten
   oder
R₁ = R₂ = Methyl-, Ethyl- oder Isopropyl- und
R₃ ein Chloratom bedeuten
   oder
R₁ = Methyl-, R₂ = Ethyl- oder Isopropyl- und
R₃ = ein Wasserstoffatom bedeuten
   oder
R₁ = Ethyl-, R₂ = Isopropyl- und
R₃ = ein Wasserstoffatom bedeuten.

Vorzugsweise wird als Methylen-bis-anilin in der ersten Stufe 4'4'-Methylenbis[(2-ethyl-6-methyl)anilin] (R₁ = CH₃, R₂ = C₂H₅, R₃ = H), 4,4'-Methylenbis[(2,6-diethyl)anilin] (R₁ = R₂ = C₂H₅, R₃ = H), 4,4'-Methylenbis[(2-isopropyl-6-methyl)anilin] (R₁ = Isopropyl-, R₂ = CH₃, R₃ = H), 4,4'-Methylenbis[(3-chlor-2,6-diethyl)anilin] (R₁ = R₂ = C₂H₅, R₃ = Cl) oder 4,4'-Methylenbis[(2,6-diisopropyl)anilin] (R₁ = R₂ = Isopropyl, R₃ = H) eingesetzt.

Zweckmässig wird die Umsetzung in der ersten Stufe stöchiometrisch mit 2 mol Maleinsäureanhydrid, gegebenenfalls in einem leichten Überschuss, bezogen auf 1 mol Methylen-bis-anilin, durchgeführt.

Die Umsetzung in der ersten Stufe erfolgt zweckmässig bei Temperaturen von 20 bis 140°C, vorzugsweise von 70 bis 90°C.

Als Lösungsmittel werden in der ersten und in der zweiten Stufe unhalogenierte aromatische Kohlenwasserstoffe angewendet. Zweckmässig werden Toluol, Xylol-Isomere, Gemische von Toluol und Xylol-Isomeren, Ethylbenzol, Cumol oder Cymol-Isomere als Lösungsmittel angewendet, vorzugsweise Xylol-Isomere oder Toluol.

Nach der ersten Reaktionsstufe wird dann direkt zur in der ersten Stufe gebildeten Amidsäure der saure Katalysator zugefügt. Zweckmässig wird der saure Katalysator in einer Menge von 0,01 bis 0,3 mol, vorzugsweise von 0,05 bis 0,1 mol, pro mol Maleinsäureanhydrid angewendet.
Als saure Katalysatoren können beispielsweise p-Toluolsulfonsäure, Methansulfonsäure, Benzolsulfonsäure oder deren Hydrate angewendet werden. Vorzugsweise wird p-Toluolsulfonsäuremonohydrat angewendet.

Die Umsetzung in der zweiten Stufe wird zweckmässig bis zur Rückflusstemperatur des Lösungsmittels oder Lösungsmittelgemisches im Bereich zwischen 90 und 140°C, vorzugsweise bei Rückflusstemperatur,durchgeführt.

Nach einer üblichen Umsetzungsdauer von 0,5 bis 4 h wird dann das Bismaleinimid-Derivat der Formel I auf fachmännisch übliche Weise isoliert.

Zweckmässig werden die Umsetzungszeit und die Temperatur in der zweiten Stufe so gewählt, dass man das gebildete Reaktionswasser mit dem beschriebenen Lösungsmittel durch azeotrope Destillation entfernt.

### Beispiel 1

### 4,4'-Methylenbis[N-(2-ethyl-6-methyl-1,4-phenylen)maleinimid]

In einem 2,5 l-Reaktionsgefäss mit Wasserabscheider, Rührer Thermometer, Rückflusskühler und Tropfrichter wurden 750 ml Xylol und 53,9 g Maleinsäureanhydrid (0,55 mol) auf 80°C erwärmt und während 30 min mit 70,6 g 4,4'-Methylenbis[(2-ethyl-6-methyl)anilin] (0,25 mol) in 250 ml Xylol versetzt. Nach 60 min Nachreaktion wurden 9,51 g p-Toluolsulfonsäure-monohydrat (0,05 mol) zugefügt und der Reaktorinhalt auf Rückflusstemperatur erwärmt. Nach 30-50 min haben sich im Wasserabscheider 8 ml wässrige Phase abgeschieden. Man liess 60 min nachreagieren und auf 20°C abkühlen, wusch mit Natriumbicarbonatlösung und entfernte aus der abgetrennten organischen Phase durch Vakuumdestillation das Lösungsmittel. Der Destillationsrückstand wurde aus Butanol umkristallisiert.
Ausbeute: Es wurden 101,5 g 4,4'-Methylenbis[N-(2-ethyl-6-methyl-1,4-phenylen)maleinimid] erhalten.
Gehalt: 99,2% (HPLC) entsprechend einer Ausbeute von 91,7%, bezogen auf eingesetztes 4,4'-Methylenbis[N-(2-ethyl-6-methyl)anilin].
Smp.: 164-165°C
- ¹H-NMR (CDCl₃, 300 MHz) δ in ppm:: 7,02 (s, 2H); 6,98 (s, 2H);
6,87 (s, 4H); 3,93 (s, 2H);
2,39 (q, 4H); 1,11 (t, 6H);
2,07 (s, 6H, J = 7,6 Hz).

### Beispiele 2 bis 5

Analog zu Beispiel 1 wurden aus den entsprechenden Methylenbisanilinen die folgenden Bismaleinimid-Derivate hergestellt:

### 4,4'-Methylenbis[N-(2,6-diethyl-1,4-phenylen)maleinimid]

Ausbeute: 85% bez. eingesetztes Methylenbisanilin
Smp.: 165-166°C
- ¹H-NMR (CDCl₃, 300 MHz) δ in ppm:: 7,04 (s. 4H); 6,84 (s, 4H);
3,99 (s, 2H); 2,38 (q, 8H);
1,11 (t, 12H).
J_{CH2,CH3}=7,6 Hz

### 4,4'-Methylenbis[N-(2-isopropyl-6-methyl-1,4-phenylen)maleinimid]

Ausbeute: 88% bez. eingesetztes Methylenbisanilin
Smp.: 195-197°C
- ¹H-NMR (CDCl₃, 300 MHz) δ in ppm:: 7,09 (s. 2H); 6,96 (s, 2H);
6,86 (s, 4H); 3,97 (s, 2H);
2,65 (sept, 2H); 1,15 (d, 12H);
2,06 (s, 6H).
J_{CH,CH3}=6,9 Hz

### 4,4'-Methylenbis[N-(3-chlor-2,6-diethyl-1,4-phenylen)maleinimid

Ausbeute: 95% bez. eingesetztes Methylenbisanilin
Smp.: 185-187°C
- ¹H-NMR (CDCl₃, 300 MHz) δ in ppm:: 6,90 (s. 2H); 6,88 (s, 4H);
4,26 (s, 2H); 2,61 (q, 4H);
1,09 (t, 6H); 2,31 (q, 4H);
1,04 (t, 6H).
J_{CH2,CH3}=7,6 Hz

### 4,4'-Methylenbis[N-(2,6-diisopropyl-1,4-phenylen)maleinmid]

Ausbeute: 76% bez. eingesetztes Methylenbisanilin
Smp.: 216-218°C
- ¹H-NMR (CDCl₃, 300 MHz) δ in ppm:: 7,06 (s. 4H); 6,87 (s, 4H);
4,07 (s, 4H); 2,60 (sept. 4H);
1,13 (d, 24H).
J_{CH,CH3}=6,9 Hz

## Patentansprüche

1. Verfahren zur Herstellung von Bismaleinimid-Derivaten der Formel 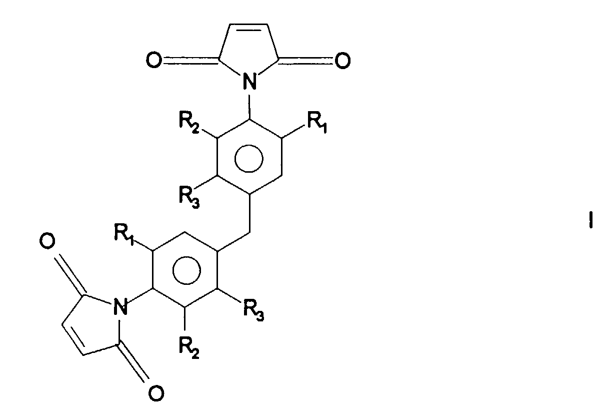 worin R₁ und R₂ gleich oder verschieden sind und eine C₁-C₄-Alkylgruppe, verzweigt oder unverzweigt, bedeuten und R₃ ein Halogen- oder Wasserstoffatom bedeutet, bei dem man in der ersten Stufe Maleinsäureanhydrid der Formel 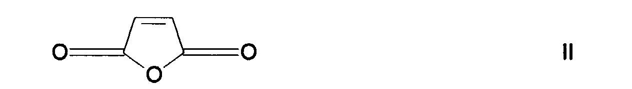 mit einem Methylenbisanilin der allgemeinen Formel 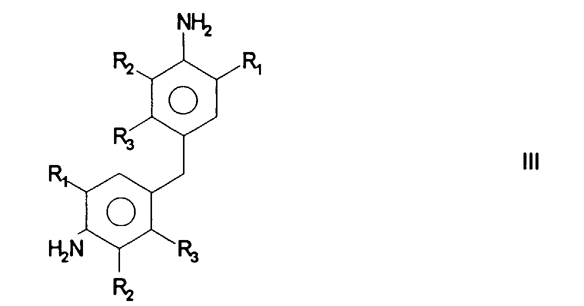 worin R₁, R₂ und R₃ die genannte Bedeutung haben in einem Lösungsmittel in die entsprechende Amidsäure überführt, und diese Amidsäure, ohne Isolierung, in der zweiten Stufe in Gegenwart eines sauren Katalysators in das Endprodukt überführt, dadurch gekennzeichnet, dass man die Umsetzung in einem unhalogenierten aromatischen Kohlenwasserstoff als Lösungsmittel durchführt.

2. Verfahren nach Patentanspruch 1, dadurch gekennzeichnet, dass man in der ersten Stufe als Methylen-bis-anilin 4,4'-Methylenbis[(2-ethyl-6-methyl)anilin], 4,4'-Methylenbis[(2,6-diethyl)anilin], 4,4'-Methylenbis[(2-isopropyl-6-methyl)anilin], 4,4'-Methylenbis[(2,6-diisopropyl)anilin] oder 4,4'-Methylenbis[(3-chlor-2,6-diethyl)anilin] einsetzt.

3. Verfahren nach einem der Patentansprüche 1 und 2, dadurch gekennzeichnet, dass man die Umsetzung in der ersten Stufe bei einer Temperatur von 20 bis 140°C durchführt.

4. Verfahren nach mindestens einem der Patentansprüche 1 bis 3, dadurch gekennzeichnet, dass man in der zweiten Stufe als sauren Katalysator p-Toluolsulfonsäure oder dessen Hydrate verwendet.

5. Verfahren nach mindestens einem der Patentansprüche 1 bis 4, dadurch gekennzeichnet, dass man die Umsetzung in der zweiten Stufe bei einer Temperatur von 90°C bis Rückflusstemperatur durchführt.

6. Verfahren nach mindestens einem der Patentansprüche 1 bis 5, dadurch gekennzeichnet, dass man in der ersten und zweiten Stufe als unhalogenierten aromatischen Kohlenwasserstoff Toluol oder Xylol-Isomere verwendet.

## Claims

1. Process for the preparation of derivatives of bismaleimides of the formula 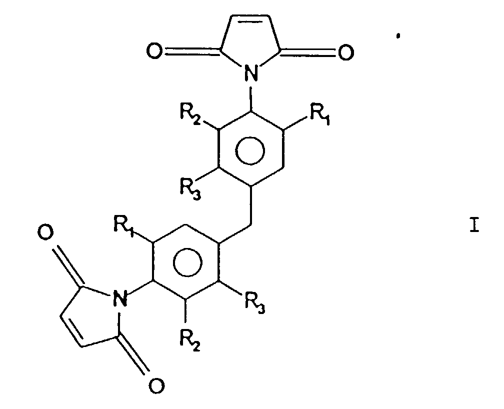 wherein R₁ and R₂ are the same or different and represent a C₁-C₄-alkyl group, branched or linear, and R₃ represents a halogen or hydrogen atom, in which process in a first step maleic anhydride of the formula 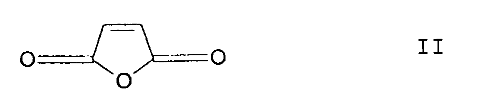 is reacted in a solvent with a methylenebisaniline of the general formula 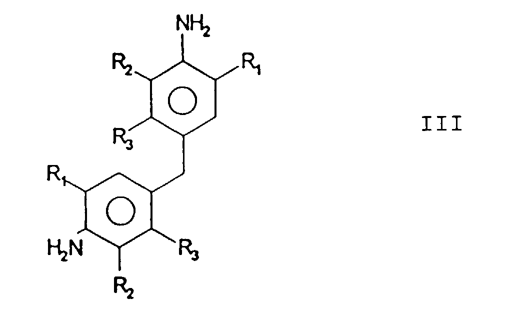 wherein R₁, R₂ and R₃ are as defined above, to form the corresponding amide acid, and said amide acid is converted, without isolation, in a second step in the presence of an acid catalyst to the final product, characterized in that the reaction is carried out in a nonhalogenated aromatic hydrocarbon as the solvent.

2. Process according to claim 1, characterized in that 4,4'-methylenebis[(2-ethyl-6-methyl)aniline], 4,4'-methylenebis[(2,6-diethyl)aniline], 4,4'-methylenebis[(2-isopropyl-6-methyl)aniline], 4,4'-methylenebis[(2,6-diisopropyl)aniline] or 4,4'-methylenebis [(3-chloro-2,6-diethyl)aniline] is used as the methylenebisaniline in the first step.

3. Process according to any one of claims 1 or 2, characterized in that in the first step the reaction is carried out at a temperature of from 20 to 140°C.

4. Process according to at least one of claims 1 to 3, characterized in that p-toluenesulfonic adid or hydrates thereof are used as the acid catalyst in the second step.

5. Process according to at least one of claims 1 to 4, characterized in that in the second step the reaction is carried out at a temperature of from 90°C to reflux temperature.

6. Process according to at least one of claims 1 to 5, characterized in that toluene or xylene isomers are used as the nonhalogenated aromatic hydrocarbon in the first and in the second step.

## Revendications

1. Procédé pour la préparation de dérivés de bismaléinimide de la formule 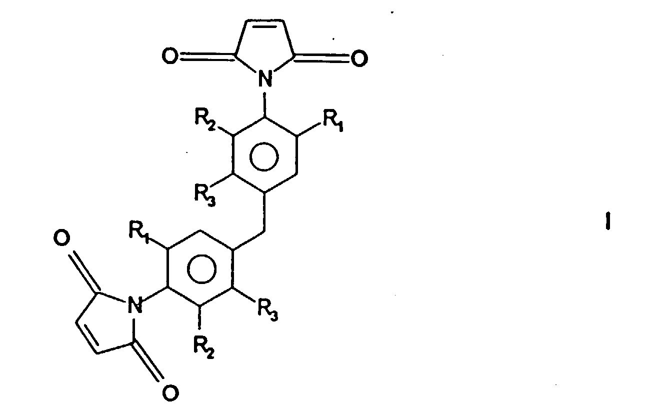 dans laquelle R₁ et R₂ sont identiques ou différents et signifient un groupe alkyle C₁-C₄, ramifié ou non ramifié, et R₃ signifie un atome d'halogène ou d'hydrogène, procédé dans lequel au cours de la première étape, on transforme l'anhydride de l'acide maléinique de la formule 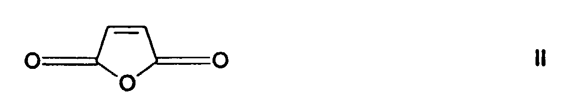 avec une méthylène-bisaniline de la formule générale 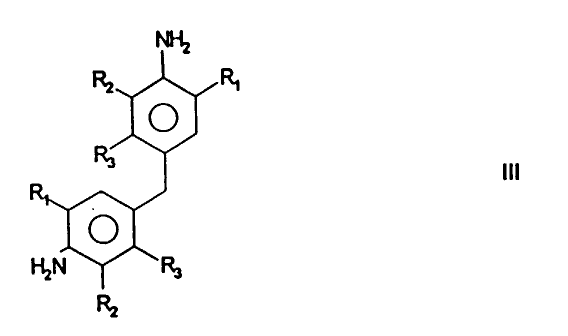 dans laquelle R₁, R₂ et R₃ ont la signification citée dans un solvant en l'acide amide correspondant, et on transforme cet acide amide, sans isolement, dans la deuxième étape en présence d'un catalyseur acide en le produit final, caractérisé en ce que l'on procède à la réaction dans un hydrocarbure aromatique non halogéné en tant que solvant.

2. Procédé selon la revendication 1, caractérisé en ce qu'au cours de la première étape, on met en oeuvre le 4,4'-méthylenbis[(2-éthyl-6-méthyl)anilin], le 4,4'-méthylènbis[(2,6-diéthyl)anilin], le 4,4'-méthylènbis[(2-isopropyl-6-méthyl)anilin], le 4,4'-méthylènbis[(2,6-diisopropyl)anilin] ou le 4,4'-méthylènbis[(3-chlor-2,6-diéthyl)anilin] en tant que méthylène-bis-aniline.

3. Procédé selon l'une des revendications 1 et 2, caractérisé en ce que l'on procède à la réaction dans la première étape à une température de 20 à 140°C.

4. Procédé selon au moins l'une des revendications 1 à 3, caractérisé en ce qu'au cours de la deuxième étape, on utilise en tant que catalyseur acide, l'acide p-toluolsulfonique ou ses hydrates.

5. Procédé selon au moins l'une des revendications 1 à 4, caractérisé en-ce qu'au cours de la deuxième étape, on procède à la réaction à une température de 90°C jusqu'à la température de reflux.

6. Procédé selon au moins l'une des revendications 1 à 5, caractérisé en ce qu'au cours de la première et de la deuxième étape, on utilise en tant qu'hydrocarbure aromatique non halogéné des isomères de xylol ou de toluol.
